# EUROPÄISCHE PATENTSCHRIFT

(11) **EP 2 964 091 B1**
(45) Veröffentlichungstag und Bekanntmachung des Hinweises auf die Patenterteilung: **24.04.2019**
(21) Anmeldenummer: 14712612.2
(22) Anmeldetag: 28.02.2014
(51) Int. Cl.: A61B 5/00, G01L 19/00, A61B 5/20, G01L 9/00

(54) **VORRICHTUNG ZUR DRUCKMESSUNG IN EINEM FLUID**
DEVICE FOR MEASURING PRESSURE IN A FLUID
DISPOSITIF DE MESURE DE PRESSION DANS UN FLUIDE

(30) Priorität: 04.03.2013 DE 102013102085
(43) Veröffentlichungstag der Anmeldung: 13.01.2016
(73) Patentinhaber: Medical Measurement Systems B.V., 7521 PV Enschede (NL)
(72) Erfinder: JENSEN, Michael, Gondy, DK-4450 Jyderup (DK); LARSEN, Kristine, 81476 München (DE); VAN GORKOM, David, 83209 Prienam Chiemsee (DE); WITTE, Jens, 81476 München (DE)
(74) Vertreter: Wittmann, Ernst-Ulrich
(86) Internationale Anmeldenummer: PCT/EP2014/053967
(87) Internationale Veröffentlichungsnummer: WO 2014/135456

(56) Entgegenhaltungen:
- EP-A2- 0 208 955
- WO-A2-99/37983
- DE-A1- 10 032 616
- DE-A1-102008 015 322
- US-A1- 2003 200 812

## Beschreibung

Die vorliegende Erfindung betrifft eine Vorrichtung zur Druckmessung in einem Fluid, insbesondere für die medizinische Diagnose und ein Messkammer zur Verwendung in der Vorrichtung zur Druckmessung in einem Fluid insbesondere im Bereich der Urodynamik und Gastroenterologie.

Im Stand der Technik ist die diagnostische Druckmessung insbesondere in der Urodynamik bekannt. U.a. bei der Druckmessung mit wassergefüllten Kathetern wird der Druck im Körper über eine Wassersäule, welche durch die Lumen der Katheter und die Druckübertragungsschläuche geführt wird, auf externe Drucksensoren geleitet. Dort misst man dann den Druck im Körper inkl. einen hydrostatischen Druck, der sich aus der Höhendifferenz der Messwertgeber zum Messort ergibt. Dieser Druckoffset wird entweder durch eine definierte Positionierung der Sensoren (Os Pubis-Höhe in der Urodynamik) oder durch elektronisches Nullsetzen kompensiert.

So offenbart die DE 100 32 616 A1 einen Druckdom mit einer Membran und einer darunter liegenden Transducermembran, die mit einem Sensor (Transducer) zur Umwandlung von Drücken in elektrische Signale verbunden ist. Der Druckdom ist mit dem darunter liegenden Gehäuse mittels Haken verbunden.

Die US 2003 200 812 A1 offenbart einen Druckdom, der an eine mit Gel gefüllte Kammer, mit einer Messmembran mit einem gegenüberliegenden Sensor, mittels Haken angekoppelt wird. Durch ein geeignet gasdichtes Membranmaterial wird verhindert, dass Luft zwischen die Membran des Druckdoms und die Messmembran gelangt.

Die DE 10 2008 015 322 A1 offenbart ein Systemelement mit einer Messkammer zur lösbaren abgedichteten Verbindung eines Messwertaufnehmers mit einem Fluidsystem. Für eine sichere Verbindung weist die Membran der Messkammer einen umlaufenden Wulst mit Löchern auf. In diese Löcher greifen Zapfen ein und sichern so die Membran gegen Abziehen.

Die WO 1999 037 983 A2 offenbart ein Verbindungselement zur Verbindung eines Messwertaufnehmers mit einem abgedichteten Fluidsystem, das einen Druckdom mit einer Membran aufweist, zur mechanischen Kopplung dieser Teile mittels einer Schnappverbindung.

Die EP 0 208 955 A2 offenbart ein Verfahren zur Herstellung eines druckübertragenden Kontaktes zwischen der Membran eines Druckdomes und dem Druckübertrager eines Messwandlers. Dazu werden Nasen des Messwandlers mittels Klammern an den Druckdom fixiert.

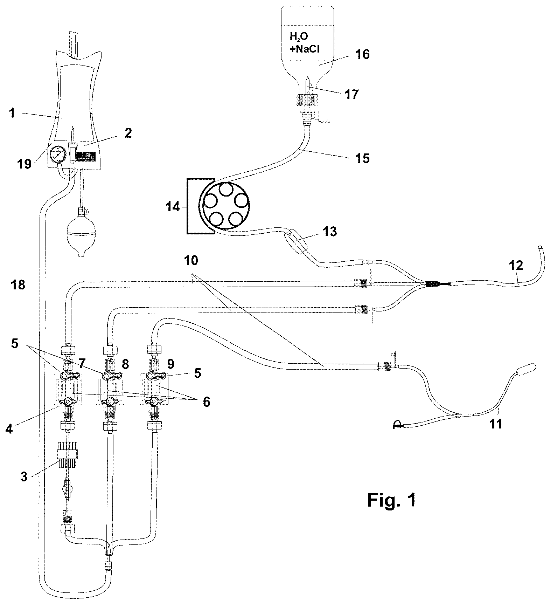

Heute bedarf eine urodynamische Untersuchung einen erheblichen Aufwand in der Vorbereitung und es wird eine erhebliche Anzahl an sterilen Einmalartikel benötigt. Ein Pumpenschlauch, ein Perfusionsschlauch, drei Druckmesswertgeber mit jeweils einem Drei- und einem Zweiwegehahn, drei Druckübertragungsschläuche, ein Transurethralkatheter mit einem Füll-sowie zwei Messvolumen und einem Rektalkatheter mit Ballon, welche alle am Messplatz aufgebaut und vorbereitet werden müssen (Figur 1).

Nachteilig ist jedoch die sehr komplizierte, fehlerträchtige und zeitraubende Handhabung. Jeder der verschiedenen Einzelschritte muss in Anwesenheit eines in den meisten Fällen unruhigen bis nervösen Patienten manuell ausgeführt werden, was auch bei erfahrenen Anwendern häufig zu Fehlern führt und es ist eine große Anzahl von einzelnen Einmalartikeln notwendig, welche häufig zu logistischen Problemen führen.

Ausgehend von diesem Stand der Technik ist es nun Aufgabe der vorliegenden Erfindung eine Vorrichtung zur Druckmessung in einem Fluid bereit zu stellen, mit welchem die im Stand der Technik bekannten Nachteile wenigstens teilweise überwunden bzw. verbessert werden.

Gelöst wird diese Aufgabe durch eine erfindungsgemäße Vorrichtung zur Druckmessung in einem Fluid gemäß Anspruch 1. Bevorzugte Ausgestaltungsformen der Vorrichtung zur Druckmessung in einem Fluid und der Messkammer sind Gegenstand der jeweiligen Unteransprüche.

Die vorliegende Erfindung umfasst eine Vorrichtung zur Druckmessung in einem Fluid, bestehend aus einem Kopplungselement, wenigstens einem Druckwandler und wenigstens eine, mit einem Fluid befüllbare Messkammer, wobei die Messkammer über eine Membran mechanisch mit der Messfläche des Druckwandlers gekoppelt ist und die Messkammer wenigstens zwei Anschlussstellen für einen Fluidstrom aufweist. Die Vorrichtung ist dadurch gekennzeichnet, dass der wenigstens eine Druckwandler in dem Kopplungselement angeordnet ist, und die wenigstens eine Messkammer zwei gegenüberliegende äußere Stege aufweist, wobei einer der Stege in eine Klemmkante des Kopplungselementes eingreift, und der andere Steg in eine Andruckwalze eingreift, wobei die Andruckwalze drehbar gelagert und kraftschlüssig mit der Kopplungseinheit verbindbar ist.

Als Fluid gemäß der vorliegenden Erfindung werden fließfähige Systeme insbesondere Flüssigkeiten betrachtet, die beispielsweise in der Medizin oder Medizintechnik verwendet werden. Solche sind beispielsweise Infusionslösungen wie Natriumchlorid Lösungen, Wasser, wässerige Lösungen, Injektionslösungen, Infusionslösungen, Nährlösungen, Elektrolytlösungen, Blut, Plasma, Gas, Luft, Kombinationen hieraus und dergleichen.

Diese Fluide werden gemäß einer weiteren besonders bevorzugten Ausführungsform in Fluidreservoiren bevorratet, die auch wiederrum im Stand der Technik wie beispielsweise in der Form von Infusionsflaschen oder Infusionsbeutel bekannt sind. Der Druckwandler ist gemäß einer bevorzugten Ausführungsform ein mechano-elektrischer Druckwandler und vorzugsweise wenigstens der Messbereich des Druckwandlers formschlüssig von der Membran bedeckt. Alternativ oder in Kombination ist der Messbereich des Druckwandlers zentrisch in Bezug auf die Grundfläche der Messkammer angeordnet
Die Messkammer hat ferner vorzugsweise eine im Wesentlichen kreisrunde Grundfläche, wobei alternativ oder in Kombination die Grundfläche offen ist und/oder die Membran die offene Grundfläche abdeckt und/oder die Membran aus einem Material hergestellt ist, welches aus einer Gruppe ausgewählt wird, welche Silikon, Latex, Kautschuk, Kombinationen hiervon und dergleichen enthält. Darüber hinaus ist die Membran gemäß einer weiteren besonders bevorzugten Ausführungsform integraler Bestandteil der Messkammer. Der Innenraum der Messkammer ist in einer weiteren bevorzugten Ausführungsform im Wesentlichen kuppelförmig gestaltet und/oder der kuppelförmige Innenraum weist im zentralen Bereich eine Abflachung auf. Die ist ferner so gestaltet, das sie ein Volumen an dem Fluid aufnehmen kann, welches zwischen von 0,1 cm³ und 10 cm³, vorzugsweise zwischen von 1 cm³ und 5 cm³ liegt und/oder die Grundfläche der Messkammer zwischen von 0,1 cm² und 5 cm², vorzugsweise zwischen von 1 cm² und 2,5 cm² groß ist.

Entsprechend einer weiteren, besonders bevorzugten Ausführungsform umfasst die erfindungsgemäße Vorrichtung zur Druckmessung in einem Fluid wenigstens zwei, vorzugsweise eine Vielzahl von Messkammern, welche vorzugsweise an einer stegfreien Außenkante miteinander direkt oder indirekt verbunden sind.

Die Messkammer und/oder das Kopplungselement sind entsprechend der vorliegenden Erfindung wenigstens abschnittsweise oder teilweise aus einem Material hergestellt wird, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

Die Anschlussstellen für den Fluidstrom, welcher in die Messkammer ein- bzw. aus der Messkammer abgeführt wird, sind gemäß einer besonders bevorzugten Ausführungsform der vorliegenden Erfindung in einem Winkel (α) von 180° bis 60°, bevorzug in einem Winkel von 180° bis 120°, besonders bevorzugt in einem Winkel (α)
von 144° bis 115°, insbesondere in einem Winkel von ca. 150° zueinander angeordnet. Darüber hinaus und alternativ sind die Anschlussstellen in einem Winkel (β) von 0° bis 60°, vorzugsweise von 12° bis 18°, vorzugsweise von weniger als 60° und insbesondere von ca. 15° zur Grundfläche der Messkammer angeordnet.

Um die Anschlussstellen möglichst einfach mit einem Schlauchsystem verbinden zu können, weisen die Anschlussstellen gemäß einer weiteren Ausführungsform einzeln oder in Kombination einen positiven oder negativen Luer-Lock-Anschluss auf.

Um die Messkammer mit dem Druckwandler zu verbinden, ist der Druckwandler in einem Kopplungselement angeordnet und die Messkammer/n werden über die äußeren Stege der Messkammer/n und der Klemmkante des Kopplungselements derart verbunden, dass über die flexible Membran mittels dem Druckwandler der Innendruck in der Messkammer bestimmbar ist. Damit die Verbindung zwischen dem Kopplungselement und der Messkammer möglichst einfach erfolgen kann, weist das Kopplungselement eine Andruckwalze auf, in welche ein Steg der Messkammer eingreift und welcher dann über eine Drehbewegung der Andruckwalze arretiert wird und gelichzeitig die notwendige Anpresskraft zur Verbindung der Messkammer mit dem Druckwandler bereitstellt. Durch diese Drehung wird vorzugsweise die Messkammer, die Membran und der Druckwandler fluiddicht bzw. für die Übertragung des Messkammerinnendrucks miteinander verbunden. Die Drehung der Andruckwalze kann hierbei händisch oder aber auch mittels eines Motors, insbesondere einen Elektromotor, einen Schrittmotor oder einen Servomotors erfolgen.

Die Aufgabe der vorliegenden Erfindung wird neben der Vorrichtung zur Druckmessung auch durch eine, mit einem Fluid befüllbaren Messkammer zur Verwendung in einer Vorrichtung zur Druckmessung mit wenigstens zwei Anschlussstellen für einen Fluidstrom gelöst. Die Anschlussstellen für die Zu- und Abführung des Fluids sind in einem Winkel (α) von 180° bis 60°, bevorzug in einem Winkel von 180° bis 120°, besonders bevorzugt in einem Winkel von 144° bis 115°, insbesondere in einem Winkel von ca. 150° zueinander angeordnet sind. Alternativ oder in Kombination können die Anschlussstellen auch in einem Winkel (β) von 0° bis 60°, vorzugsweise von 12° bis 18°, vorzugsweise von weniger als 60° und insbesondere von ca. 15° zur Grundfläche der Messkammer angeordnet sind. Wie bereits in Bezug auf die Vorrichtung dargestellt ist entsprechend einer weiteren, bevorzugten Ausführungsform wenigstens eine der Anschlussstellen mit einem positiven oder negativen Luer-Lock-Anschluss versehen.

Als Grundfläche wird hierbei insbesondere die Fläche verstanden, welche zum Druckwandler der Vorrichtung zur Druckmessung bei der Messung ausgerichtet wird. Die Anschlussstellen sind ferner und entsprechend einer besonders bevorzugten Ausführungsform in einem Abstand zur Grundfläche von 2 bis 10 mm, bevorzugt von 4 bis 8 mm und besonders bevorzugt von 6 mm angeordnet und alternativ oder in Kombination weist wenigstens eine der Öffnungen der Anschlussstellen im Innenraum der Messkammer wenigstens abschnittsweise eine der Grundfläche zugewandte Ausformung auf.

Der Innenraum der Messkammer weist ferner vorzugsweise eine im Wesentlichen kreisrunde Grundfläche auf, wobei diese offen ist und/oder die Membran die offene Grundfläche abdeckt. Die Membran ist gemäß einer weiteren besonders bevorzugten Ausführungsform aus einem Material hergestellt, welches aus einer Gruppe ausgewählt wird, welche Silikon, Latex, Kautschuk, Kombinationen hiervon und dergleichen enthält. Der Innenraum der Messkammer kann darüber hinaus auch im Wesentlichen kuppelförmig gestaltet sein und/oder im zentralen Bereich abgeflacht werden. Ferner umfasst die Messkammer an wenigstens zwei gegenüberliegenden Außenkanten Stege auf, wobei alternativ oder in Kombination an wenigstens einer der stegfreien Kanten wenigstens eine weitere Messkammer angeordnet ist.

Die vorliegende Erfindung umfasst ausdrücklich auch die Verwendung der vorbeschriebenen Messkammer für eine Vorrichtung zur Druckmessung von Fluid bzw. Fluiden, wobei die Messkammer und die Vorrichtung u.a. und bevorzugt in der Medizin oder der Medizintechnik, insbesondere im Bereich der Urodynamik und Gastroenterologie, insbesondere zur Blasen-, Rektal- und Urethraldruckmessung eingesetzt werden.

Nachfolgend wird die Erfindung anhand eines bevorzugten Ausführungsbeispiels beschrieben, wobei darauf hingewiesen wird, dass die Erfindung nicht auf die hier dargestellte Ausführung beschränkt, sondern vielmehr auch entsprechende Abwandlungen im Sinne der vorliegenden Erfindung sind.

Dabei zeigen:
- Figur 1: den Aufbau eines Messsystem gemäß dem Stand der Technik;
- Figur 2: den Aufbau eines Druckmesssystems gemäß der vorliegenden Erfindung;
- Figur 3: eine Detailansicht des Pumpenschlauchs für das erfindungsgemäße Druckmesssystems;
- Figur 4: eine Explosionsansicht für die Zusammenstellung der Druckdomkassette mit den Druckmesssensoren;
- Figuren 5a bis 5d: Detaildarstellungen der Domkassette und deren Verriegelungsanordnung auf den Druckmesssensoren;
- Figur 6: Messprotokoll einer automatischen Entlüftung und Nullstellung des erfindungsgemäßen Druckmesssystems.

In Figur 1 ist schematisch der Aufbau einer Vorrichtung zur Druckmessung mit wassergefüllten Einmalkathetern dargestellt. Dabei ist neben dem Beutel mit einer Infusionslösung 1 und einer Druckmanschette 2, ein Druckminderer 3 und Zweiwegehähne 4 vor den Drucksensoren 6 dargestellt. Im Anschluss hieran sind Dreiwegehähne 5 angeordnet und die entsprechenden Leitungen werden als Pᵤᵣₐ 7, Pᵥₑₛ 8 und P_{abd} 9 bezeichnet. Über die Druckübertragungsschläuche 10 wird die Infusionslösung aus dem Beutel 1 zu dem Rektalkatheter 11 und den UDP-Katheter 12 geleitet. Darüberhinaus wird zum UDP-Katheter 12 aus der Flasche 16 mit z.B. einer Kochsalzlösung über die Rollenpumpe 14 und die Tropfkammer 13 die Kochsalzlösung aus der Flasche 16 definiert zugeführt.

Hiermit wird für die Vorbereitungs- und Messprozedur wie folgt verfahren:
a) Der Pumpenschlauch 15 wird in die Pumpe 14 eingelegt und der Dorn 12 des Schlauchs in den Flaschenstopfen eingesetzt. Die Pumpe 14 wird eingeschaltet bis die Tropfenkammer 13 der Flasche 16 halb gefüllt und der Schlauch komplett luftblasenfrei mit Kochsalzlösung gefüllt ist.
b) Die drei Druckmesswertgeber 6 werden in den Halterungen platziert und mit dem Perfusionsschlauch 18 verbunden - zwischen den Perfusionsschlauch und einen Druckmesswertgeber wird ein Durchflußminderer 3 eingesetzt. Der Dorn 19 des Perfusionsschlauchs 18 wird in die Wasserbeutel 1 eingesteckt.
c) Die Druckübertragungsschläuche 10 werden mit den Druckmesswertgebern 6 verbunden.
d) Alle Zwei- 4 und Dreiwegehähne 5 werden geschlossen und die Druckmanschette 2 wird aufgepumpt um die Wasserbeutel 1 unter Druck zu setzen.
e) Zum Entlüften der Druckübertragungslinien werden die Zwei- 4 und Dreiwegehähne 5 der Drucktransducer auf "offen" gestellt und blasenfrei bis an die Spitze mit Wasser gefüllt und anschließend der Zweiwegehahn 2 wieder geschlossen. Diese Prozedur muss für alle drei Druckübertragungslinen einzeln durchgeführt werden.
f) Die Dreiwegehähne 5 werden jetzt einzeln in "90 grad"- Position gedreht um die Druckkanäle per Tastendruck elektronisch auf Atmosphäre abzugleichen. Die Dreiwegehähne 5 werden anschließend wieder auf die Position "offen " gedreht.
g) Die beiden Katheter 11, 12 werden in Urethra und Rektum des Patienten platziert und an die Druckübertragungslinien sowie den Pumpenschlauch 10 angeschlossen.
h) Die Dreiwegehähne 5 müssen jetzt noch mal einzeln geöffnet um die beiden Lumen des Transurethralkathers 12 bis an die Spitze zu entlüften und den Ballon des Rektalkatheters 11 zu füllen.
i) Die gemessene Drücke werden überprüft und wenn notwendig per Software auf Null gestellt. Das System ist jetzt messbereit.

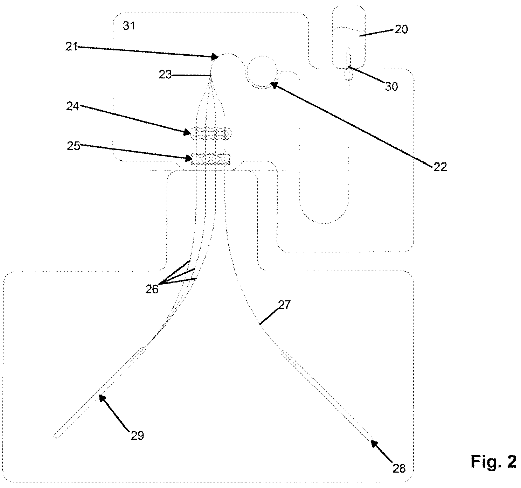

In Figur 2 ist der Aufbau eines erfindungsgemäßen Druckmesssystems dargestellt.

Dieses zeigt mit dem Bezugszeichen 21 den Pumpenschlauch des zu der Druckdomkassette 25 mit der Luer-Locks für den Anschluss von Kathetern 28 und 29 führt. Ferner ist der Einstichdorn 30 für Standard-Infusionbeutel 20 dargestellt. Mit dem Bezugszeichen 29 ist der Transurethraler und mit dem Bezugszeichen 28 der Rektalkatheter (ggf. auch beliebig viele Katheter oder Messvolumen), bezeichnet, welche mit dem Fluid über die Leitungen 26 (drei Stufen) und 27 versorgt werden. Hieran anschließend ist der Verriegelungsmechanismus der Domkassette 25 angeordnet. Im Bereich des Bezugszeichens 24 sind vorliegend vier (ggf. beliebig viele) Schlauchklemmen mit den Zuständen "offen", "geschlossen" und "perfundiert" angeordnet. Mit dem Bezugszeichen 22 ist eine Rollpumpe zur Beförderung des Mediums durch das Schlauchsystem dargestellt. Das System umfasst ferner ein Steuerungssystem 31 mit vollautomatischen Algorithmus zur Entlüftung des Schlauchsystems mit Kathetern, Auffindung von Ruhedrücken und Nullstellung des Messsystems unter Ausnutzung der Funktionalität der Einzelkomponenten.

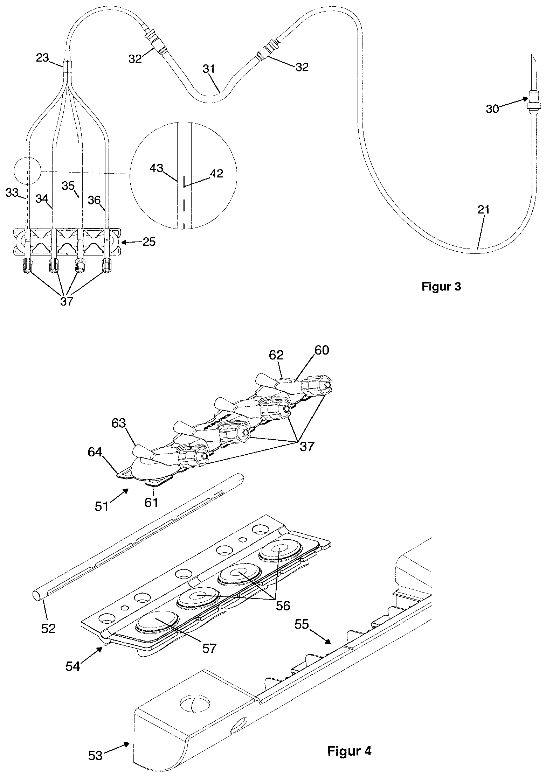

Im Pumpenschlauch - wie in Figur 3 gezeigt - sind der Infusionsschlauch 21, die Entlüftungsschläuche 34 bis 36 für die drei - ggf. beliebig viele - Druckkanäle und die Domkassette 25 integriert. Zudem besitzt der Pumpen- bzw. Infusionsschlauch 21 einen Anstichdorn 30 für einen Infusionsbeutel und Luer-Locks 37 für den Anschluss der Katheter. Bei der Verwendung des Systems mit einer Rollenpumpe weist das Schlauchsystem in diesem Bereich einen geeigneten kompressiblem Schlauchabschnitt 31 auf, der durch entsprechende Verbinder 32 in das Schlauchsystem integriert ist. Über den Verteiler 23 wird das Schlauchsystem auf die hier dargestellten vier Schläuche 33 bis 36 aufgeteilt. Der Schlauch 33 umfasst neben dem eigentlichen Schlauch 42 auch den Abstandshalter 43, wie dies in der Detaildarstellung der Fig. 3 zu erkennen ist.

Besonders vorteilhaft ist bei der vorliegenden Erfindung die Integration von drei - ggf. beliebig vielen - Domen in eine Domkassette und die gemeinsame Kopplung der Kanäle an die Sensoren über eine Andruckwalze und Klemmkante. Hierbei wirken die auf Andruckwalzen- und Klemmkantenseite in die Domkassette integrierten Stege als Federelemente, die den benötigten Anpressdruck der Membrane, die vorzugsweise auf den Bodenabschnitt der Druckdome angeordnet sind, auf die Sensoren herstellen.

Diese Konstruktion hat große Vorteile gegenüber der konventionellen Lösung mit Einzeldomen: Die Herstellung ist sehr viel günstiger und die Handhabung wird extrem vereinfacht. Statt wie bei den bisherigen Lösungen jeden Dom einzeln auf seinen Sensor zu stecken, genügt das Einlegen der Kassette und ein anschließender Knopfdruck mit dem die Anpresswalze über einen Aktuator in die "geschlossen" Position gedreht wird. Die vormals hohe Anzahl von benötigten, sterilen Einzelkomponenten ist insbesondere auf ein einziges Einmalprodukt reduziert.

Da in vielen Anwendungsgebieten die Sensoren oberhalb der Messorte im Körper platziert sein können, entstehen wegen der hydrostatischen Kraft der Wassersäule auch negative Drücke (niedriger als Atmosphärendruck) im Dom, die dann nicht durch Druck auf die Sensorfläche, sondern durch Zug gemessen werden. Zur Herstellung des dazu benötigten Saugeffektes müssen die Kontaktierungen zwischen Membranen und Sensoren komplett luftdicht ausgelegt sein. Dazu benötigt man einen Anpressdruck.

Figur 4 zeigt eine Explosionsansicht für die Zusammenstellung der Druckdomkassette 51 mit den Druckmesssensoren 54. Hierbei ist neben der Domkassette 51, die Andruckwalze 52, die Klemmkante 55 aufweisende Aufnahme 53 zu erkennen. Ferner sind mit dem Bezugszeichen 56 die Positionen im Bezug auf die Domkassette 51 dargestellt, die mit einem Druckmesswertgeber ausgestattet sind. In der Position 57 ist kein Druckmesswertgeber vorgesehen.

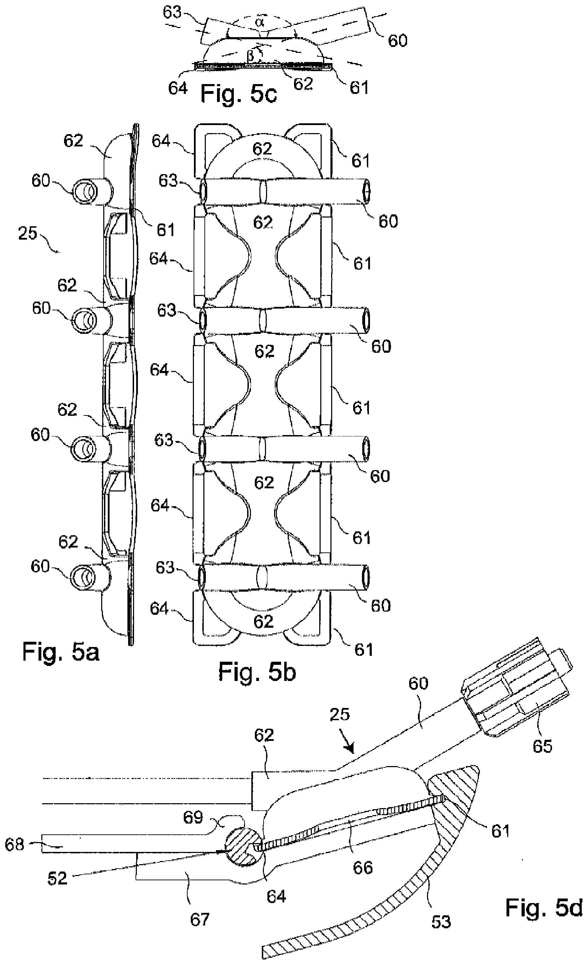

In den Figuren 5a bis 5d sind Detaildarstellungen der Domkassette 25 und deren Verriegelungsanordnung auf den Druckmesssensoren gezeigt. Dabei ist die Figur 5a eine Seitenansicht der Domkassette 25 dargestellt, bei welcher die Anschlüsse 60, der Andrucksteg 61 und Teile der Fluiddome 62 zu erkennen sind. In Figur 5b ist die Draufsicht der Domkassette 25 dargestellt, bei welcher zusätzlich die weiteren Anschlüsse 63 und den hinteren Andrucksteg 64 zu erkennen sind. Figur 5c ist eine weitere Seitenansicht, bei welcher neben den beiden Anschlüssen 60 und 63, auch die beiden Andruckstege 61 und 64 und die Winkelanordnung der Zuführungen 60 und 63 zueinander mit dem Winkel α und zum Boden mit dem Winkel β gezeigt sind.

Figur 5d zeigt die Anordnung der Domkassette 25 auf den Druckmesswertgebern, wobei die Domkassette 25 über den Andrucksteg 61 in der Aufnahme 53 ausgerichtet und mittels der Anpresswalze 52 und deren axialen Schlitzung in Kombination mit dem Verdrehen fixiert wird. Die Aufnahme 53 umfasst hierbei auch die Führung 69 und 68, als direkter oder indirekter Bestandteil der Aufnahme, die auch in den entsprechenden Positionen die Druckmesswertgeber (hier nicht dargestellt) umfasst. Neben der Aufnahme sind in dieser Darstellung auch die Anschlüsse 60 und 63 zu erkennen, wobei der Anschluss 60 auch einen Luer-Verschluss 65 aufweist.

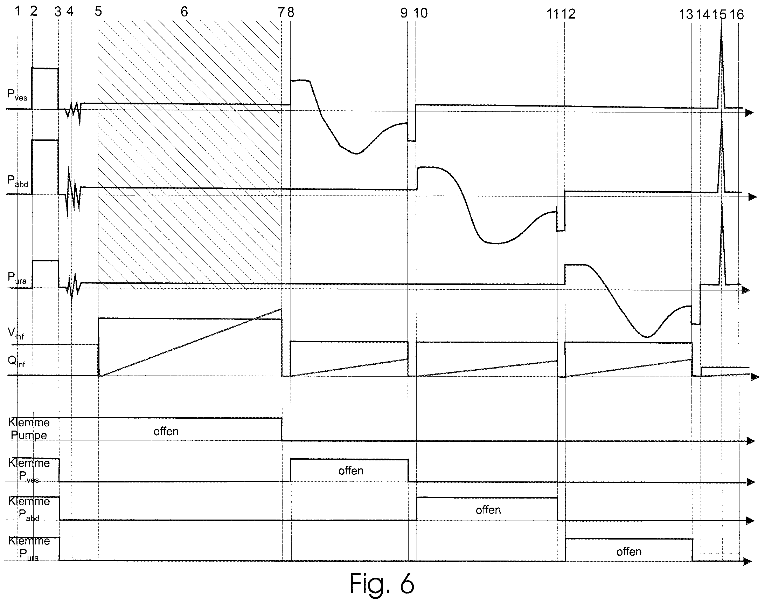

In Figur 6 ist über die Verfahrensschritte der Druckverlauf an den entsprechenden Positionen bzw. die Stellposition der Bauteile wiedergegeben. Dabei steht Pᵤᵣₐ, Pᵥₑₛ und P_{abd} für den Druck in den entsprechenden Druckdomen, V_{inf} und Q_{inf} für das Infusionsvolumen und den Fluidstrom und Klemme-Pumpe, Klemme-Pᵤᵣₐ, Klemme-P_{abd} und Klemme-Pᵥₑₛ für die Schaltposition der entsprechenden Schlauchklemme. Das Verfahren wird nachfolgend beispielhaft beschrieben:
1) Der Benutzer legt die Domkassette 25 des Pumpenschlauchs 21 in das Gerät ein und führt den Einstichdorn 30 in den Infusionsbeutel 20 ein.
2) Der Verriegelungsmechanismus wird geschlossen, wodurch durch den Anpressdruck der Dome 25 auf die Sensoren ein Druckoffset entsteht.
3) Der Druckoffset wird automatisch nach einer Materialentspannungszeit von 5-10s ausgeglichen (zu Null gesetzt). Gleichzeitig werden die Schlauchklemmen 24 der Druckkanäle 26 und 27 geschlossen.
4) Der Benutzer platziert den transurethralen Katheter 29 in die Blase und den rektalen Katheter 28 in das Rektum des Patienten (falls nicht schon vorher geschehen) und verbindet den Pumpenschlauch 21 sowie die Druckübertragungsschläuche mit den Luer-Locks der Domkassette 25. Ab diesem Zeitpunkt kann der Benutzer die korrekte Platzierung der Katheter 28,29 anhand der am Gerät angezeigten Ruhedruckwerte, die jetzt über die Luftsäule der Druckübertragungsschläuche und Katheterlumen gemessen werden, überprüfen.
5) Wenn die Ruhedrücke plausible Werte zeigen, startet der Benutzer die vollautomatische Entlüftungs- und Nullstellungsprozedur per Tastendruck am Gerät, bei der zunächst die gesamte Infusionslinie entlüftet wird.
6) Im schraffiert dargestellten Zeitintervall messen alle Druckkanäle den Ruhedruck am jeweiligen Messort über die Luftsäule. Um zufällige Schwankungen durch Bewegung des Patienten oder Störungen zu minimieren wird der durchschnittlich gemessene Wert aller Kanäle während dieses Zeitraums berechnet und später als Ruhedruckwert zur Nullstellung verwendet.
7) Da das Volumen der Infusionslinie (Schlauch plus Fülllumen des Katheters) exakt bekannt ist und die beförderte Menge des Füllmediums durch den Volumentransducer bzw. Umdrehungen des Rollenrades der Pumpe 22 ständig gemessen wird, kann die Entlüftung vollautomatisch beendet werden, wenn die Wassersäule den Fülllumenausgang erreicht hat. Die Pumpenschlauchklemme 24 wird geschlossen.
8, 10, 12) Die Entlüftung der Druckübertragungsschläuche geschieht dann sequentiell immer nach dem gleichen Schema. Die jeweilige Schlauchklemme wird geöffnet, während alle anderen geschlossen sind und die Pumpe befördert das Füllmedium in definierter Füllgeschwindigkeit durch die jeweilige Druckübertragungslinie. Zu beachten ist hierbei, dass während des Befüllens wegen der sich aufbauenden Wassersäule zunehmend eine hydrostatische und zusätzlich eine dynamische Komponente hinzukommt, nicht mehr der tatsächliche Druck am Messort ermittelt wird..
9, 11, 13) Da die Volumina der Druckübertragungslinien bekannt sind und die Füllmenge ständig gemessen wird, kann der Entlüftungsvorgang vollautomatisch beendet werden, wenn die Wassersäule die Messlumenausgänge erreicht hat. Die Pumpe 22 wird gestoppt und die jeweilige Schlauchklemme 24 geschlossen. In diesem Moment misst das Gerät den momentanen Druck am Messort im Körper plus einen sich aus dem nicht bekannten Höhenunterschied zwischen Messort und Sensor ergebenden hydrostatischen Druck. Dieser wird jetzt vollautomatisch herausgerechnet, indem man softwaremäßig den jeweiligen Messkanal auf den in (6) über Luftsäule gemessenen Ruhedruck setzt.
14, 15) Die korrekte Platzierung der Katheter wird in einem letzten Schritt überprüft, indem man den Patienten Husten lässt und die dadurch entstehenden Druckspitzen vergleicht. Dazu werden die Schlauchklemmen 24 aller Kanäle, die Perfusion für die Messung benötigen (Pura in der Urodynamik), automatisch in die Perfusionstellung und die Pumpe 22 auf Perfusionsgeschwindigkeit gestellt.
16) Die Vorbereitungsphase ist abgeschlossen und die eigentliche Messung kann beginnen.

## Patentansprüche

1. Vorrichtung zur Druckmessung in einem Fluid,
bestehend aus einem Kopplungselement (25),
wenigstens einem Druckwandler (56),
wenigstens einer, mit einem Fluid befüllbaren, Messkammer (62), wobei die Messkammer (62) über eine Membran (66) mechanisch mit der Messfläche des Druckwandlers (56) gekoppelt ist und die Messkammer (62) wenigstens zwei Anschlussstellen (60, 63) für einen Fluidstrom aufweist, und
eine Andruckwalze (52), mit der die Messkammer (62) arretiert wird,
**dadurch gekennzeichnet, dass**
der wenigstens eine Druckwandler (56) in dem Kopplungselement (25) angeordnet ist, und
die wenigstens eine Messkammer (62) zwei gegenüberliegende äußere Stege (61, 64) aufweist, wobei einer der Stege (61) in eine Klemmkante (53) des Kopplungselementes (25) eingreift, und der andere Steg (64) in die Andruckwalze (52) eingreift, wobei die Andruckwalze (52) drehbar gelagert und kraftschlüssig mit der Kopplungseinheit (51) verbindbar ist.

2. Vorrichtung nach Anspruch 1, **dadurch gekennzeichnet, dass**
die Anschlussstellen in einem Winkel (a) von 180° bis 60°, bevorzugt in einem Winkel von 180° bis 120°, besonders bevorzugt in einem Winkel von 144° bis 115°, insbesondere in einem Winkel von ca. 150°, zueinander angeordnet sind.

3. Vorrichtung nach Anspruch 1, **dadurch gekennzeichnet, dass**
die Anschlussstellen in einem Winkel (β) von 0° bis 60°, vorzugsweise von 12 bis 18°, vorzugsweise von weniger als 60° und insbesondere von ca. 15° zur Grundfläche der Messkammer angeordnet sind.

4. Vorrichtung nach einem der vorstehenden Ansprüche, **dadurch gekennzeichnet, dass**
der Innenraum der Messkammer (62) eine im Wesentlichen kreisrunde Grundfläche aufweist.

5. Vorrichtung nach einem der vorstehenden Ansprüche, **dadurch gekennzeichnet, dass**
die Grundfläche offen ist und/oder die Membran (66) die offene Grundfläche abdeckt und/oder die Membran (66) aus einem Material hergestellt ist, welches aus einer Gruppe ausgewählt wird, welche Silikon, Latex, Kautschuk, Kombinationen hiervon und dergleichen enthält.

6. Vorrichtung nach einem der vorstehenden Ansprüche, **dadurch gekennzeichnet, dass**
der Druckwandler (54) ein mechano-elektrischer Druckwandler ist und vorzugsweise wenigstens der Messbereich des Druckwandlers (54) formschlüssig von der Membran (66) bedeckt wird und/oder der Messbereich des Druckwandlers (54) zentrisch in Bezug auf die Grundfläche der Messkammer (62) angeordnet ist.

7. Vorrichtung nach einem der vorstehenden Ansprüche, **dadurch gekennzeichnet, dass**
durch Drehen der Andruckwalze die Messkammer (62), die Membran (66) und der Druckwandler (54) fluiddicht miteinander verbunden werden.

8. Vorrichtung nach einem der vorstehenden Ansprüche, **dadurch gekennzeichnet, dass**
die Messkammer (62) ein Volumen von zwischen 0,1 cm³ und 10 cm³, vorzugsweise von zwischen 1 cm³ und 5 cm³ und/oder eine Grundfläche von zwischen 0,1 cm² und 5 cm², vorzugsweise von zwischen 1 cm² und 2,5 cm² hat.

9. Vorrichtung nach einem der vorstehenden Ansprüche, **dadurch gekennzeichnet, dass** wenigstens zwei Messkammern (62) an stegfreien Außenkanten miteinander verbunden sind.

10. Vorrichtung nach einem der vorstehenden Ansprüche, **dadurch gekennzeichnet, dass**
das Kopplungselement (25) und/oder die Messkammer (62) wenigstens abschnittsweise oder teilweise aus einem Material hergestellt wird, das aus einer Gruppe ausgewählt ist, welche duro- und thermoplastische Kunststoffe und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

## Claims

1. A device for measuring pressure in a fluid,
comprising a coupling element (25),
at least one pressure transducer (56),
at least one measurement chamber (62) which can be filled with a fluid, wherein the measurement chamber (62) is mechanically coupled to the measurement surface of the pressure transducer (56) by a membrane (66) and the measurement chamber (62) has at least two connection points (60, 63) for a fluid flow, and
a pressing roller (52) with which the measurement chamber (62) is stopped, **characterized in that**
the at least one pressure transducer (56) is arranged in the coupling element (25) and the at least one measurement chamber (62) has two outer webs (61, 64) positioned opposite each other, wherein one of the webs (61) engages in a clamping edge (53) of the coupling element (25) and the other web (64) engages in the pressing roller (52), wherein the pressing roller (52) is held in a rotatable manner and can be force-fitted with the coupling unit (51).

2. The device according to claim 1, **characterized in that**
the connection points are arranged at an angle (α) of 180° to 60°, preferably at an angle of 180° to 120°, particularly preferably at an angle of 144° to 115°, and more particularly at an angle of about 150° with regard to each other.

3. The device according to claim 1, **characterized in that**
the connection points are arranged at an angle (β) of 0° to 60°, preferably 12 to 18°, preferably less than 60°, and more particularly about 15° with regard to the base area of the measurement chamber.

4. The device according to any preceding claim, **characterized in that**
the interior of the measurement chamber (62) has an essentially circular base area.

5. The device according to any preceding claim, **characterized in that**
the base area is open and/or the membrane (66) covers the open base area and/or the membrane (66) is made of a material selected from a group which contains silicone, latex, rubber, combinations thereof, and the like.

6. The device according to any preceding claim, **characterized in that**
the pressure transducer (54) is an electromechanical pressure transducer and preferably at least the measuring area of the pressure transducer (54) is covered by the membrane (66) in a form-fitted manner and/or the measuring area of the pressure transducer (54) is arranged centrically in relation to the base area of the measurement chamber (62).

7. The device according to any preceding claim, **characterized in that**
through turning the pressing roller, the measurement chamber (62), the membrane (66) and the pressure transducer (54) are connected to each other in a fluid-tight manner.

8. The device according to any preceding claim, **characterized in that**
the measurement chamber (62) has a volume of between 0.1 cm³ and 10 cm³, preferably between 1 cm³ and 5 cm³, and/or a base area of between 0.1 cm² and 5 cm², preferably between 1 cm² and 2.5 cm².

9. The device according to any preceding claim, **characterized in that**
at least two measurement chambers (62) are connected to each other at web-free outer edges.

10. The device according to any preceding claim, **characterized in that**
the coupling element (25) and/or the measurement chamber (62) are at least in sections or partially made of a material selected from a group that includes duroplastics and thermoplastics, and more particularly polyphenylene sulfide, polypropylene, poly(1-butene), polyvinyl chloride, polyvinylidene chloride, polymethyl metaacrylate, polyacrylonitrile, polystyrene, polyacetal, polyvinyl alcohol, polyvinyl acetate, ionomers, fluoroplastic, polyethylene, polyamide, and more particularly a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, thiourea resin, melamine resin, alkyl resin, allyl resin, silicone, polyimide, polybenzimidazole, epoxy resin, casein plastic, cross-linked polyurethane, unsaturated polyester resin, acrylonitrile butadiene styrene, acrylester styrene acrylonitrile, metals such as stainless steel, aluminum, combinations thereof, and the like.

## Revendications

1. Dispositif en vue de la mesure de la pression dans un fluide,
se composant d'un élément de couplage (25),
d'au moins un convertisseur de pression (56),
d'au moins une chambre de mesure remplissable par un fluide (62), la chambre de mesure (62) étant accouplé via une membrane (66) de manière mécanique avec la surface de mesure du convertisseur de pression (56) et la chambre de mesure (62) présentant au moins deux points de jonction (60, 63) pour un courant de fluide, et
d'un cylindre de compression (52), avec lequel la chambre de mesure (62) est bloquée, **caractérisé en ce**
**qu'**au moins l'un des convertisseurs de pression (56) est disposé dans l'élément de couplage (25), et
**qu'**au moins l'une des chambres de mesure (62) présente deux entretoises extérieures opposées (61, 64), une des entretoises (61) s'engrenant dans une arête de serrage (53) de l'élément de couplage (25) et l'autre entretoise (64) s'engrenant dans le cylindre de compression (52), le cylindre de compression (52) étant monté sur paliers tournants et reliable mécaniquement avec l'unité de couplage (51).

2. Dispositif selon la revendication 1, **caractérisé en ce que**
les points de jonction sont disposés les uns par rapport aux autres dans un angle (a) de 180° à 60°, de préférence dans un angle de 180° à 120°, de préférence particulière dans un angle de 144° à 115°, notamment dans un angle d'env. 150°.

3. Dispositif selon la revendication 1, **caractérisé en ce que**
les points de jonction sont disposés dans un angle (β) de 0° à 60°, de préférence de 12 à 18°, de préférence de moins de 60° et notamment d'env. 15° par rapport à la surface de base de la chambre de mesure.

4. Dispositif selon une quelconque des revendications précédentes, **caractérisé en ce que**
l'intérieur de la chambre de mesure (62) présente pour l'essentiel une surface de base circulaire.

5. Dispositif selon une quelconque des revendications précédentes, **caractérisé en ce que**
la surface de base est ouverte et/ou la membrane (66) couvre la surface de base ouverte et/ou la membrane (66) est fabriquée à partir d'une matière qui est sélectionnée à partir d'un groupe qui contient de la silicone, du latex, du caoutchouc, des combinaisons de ceux-ci et similaires.

6. Dispositif selon une quelconque des revendications précédentes, **caractérisé en ce que**
le convertisseur de pression (54) est un convertisseur de pression mécano-électrique et de préférence au moins la plage de mesure du convertisseur de pression (54) est couverte mécaniquement par la membrane (66) et/ou la plage de mesure du convertisseur de pression (54) est disposée de manière centrée par rapport à la surface de base de la chambre de mesure (62).

7. Dispositif selon une quelconque des revendications précédentes, **caractérisé en ce que,**
en tournant le cylindre de compression, la chambre de mesure (62), la membrane (66) et le convertisseur de pression (54) sont reliés les uns avec les autres de manière étanche aux liquides.

8. Dispositif selon une quelconque des revendications précédentes, **caractérisé en ce que**
la chambre de mesure (62) possède un volume d'entre 0,1 cm³ et 10 cm³, de préférence d'entre 1 cm³ et 5 cm³ et/ou une surface de base d'entre 0,1 cm² et 5 cm², de préférence d'entre 1 cm² et 2,5 cm².

9. Dispositif selon une quelconque des revendications précédentes, **caractérisé en ce**
**qu'**au moins deux chambres de mesure (62) sont reliées l'une avec l'autre sur les bords extérieurs non nervurés.

10. Dispositif selon une quelconque des revendications précédentes, **caractérisé en ce que**
l'élément de couplage (25) et/ou la chambre de mesure (62) est fabriqué au moins par section ou partiellement en une matière qui est sélectionnée à partir d'un groupe qui comporte des duroplastiques et des thermoplastiques et notamment du polysulfure de phénylène, du polypropylène, du poly-1-butylène, du chlorure de polyvinyle, du chlorure de polyvinylidène, du polyméthacrylate de méthyle, du polyacrylonitrile, du polystyrène, du polyacétal, de l'alcool polyvinylique, du polyacétate de vinyle, des ionomères, du plastique fluoré, du polyéthylène, du polyamide, notamment un polyamide aromatique partiel, du polycarbonate, du polyester, du polyphénylène-oxyde, du polysulfone, de l'acétal polyvinylique, du polyuréthane, et du polyéther chloré, du nitrate de cellulose, de l'acétate de cellulose, de l'éther de cellulose, de la résine phénolique, de la résine d'urée, de la résine thio-urée, de la résine de mélamine, de la résine d'alcoyle, de la résine allylique, de la silicone, du polyimide, du polybenzimidazole, de la résine époxy, du plastique à la caséine, du polyuréthane réticulé, de la résine polyester non saturée, de l'acrylonitrile-butadiène-styrène, de l'ester acrylique-styrène-acrylonitrile, des métaux comme par exemple l'inox, l'aluminium, des combinaisons de ceux-ci et des produits similaires.
